# EUROPEAN PATENT APPLICATION

(11) **EP 1 974 714 A2**
(43) Date of publication of application: **01.10.2008**
(21) Application number: 08006470.2
(22) Date of filing: 31.03.2008
(51) Int. Cl.: A61K 6/093, A61K 6/10

(54) **Silicone composition**

(30) Priority: 30.03.2007 JP 2007091348
(71) Applicant: GC Corporation, Itabashi-ku Tokyo 174-8585 (JP)
(72) Inventor: Kamohara, Hiroshi, Tokyo (JP)
(74) Representative: Müller-Boré & Partner Patentanwälte

(57) **Abstract**

To provide a silicone composition which does not adhere on a set material mainly including silicone, such as a silicone impression material used in the dental operation field, when being built on the set material mainly including silicone, the silicone composition includes (A) 100 weight parts of organopolysiloxane having at least two aliphatic unsaturated hydrocarbons in one molecule, (B) 0.1 to 50 weight parts of organohydrogenpolysiloxane having at least three hydrogen atoms, which directly bond to a silicon atom, in one molecule and including SiO₂ units, (C) 0.1 to 50 weight parts of fine silica powder having a BET specific surface area of 50 to 500 m²/g and being subjected to a hydrophobic treatment on the surface thereof, and 10 to 800 ppm of a silicone-soluble platinum compound with respect to 100 weight parts of the total of the (A) and (B) components.

## Description

The present invention relates to a silicone composition which does not adhere to a set material mainly including silicone even when being set on the set material mainly including silicone.

Particularly, when a dental implant is produced, the position or shape of oral mucosa is desirably reproduced on a residual ridge model, and thus a dental implant may be designed, produced, or adjusted by forming a rubber-like set body as a model of the mucosa on a residual ridge model produced with gypsum. A material used in this case is called as a gum (gingiva) model material, and a silicone composition or a polyether composition like a material conventionally used mainly as a dental impression material is used (for example, refer to Unexamined Japanese Patent Publication No. 07-112911, Unexamined Japanese Patent Publication No. 10-167923).

As for a method how to use a gum model material, that is, a method for reproducing a gum on a gypsum model, a gypsum model is produced by the steps of taking an impression in an oral cavity with a silicone impression material (a material mainly including silicone) according to an ordinary method, building a gum model material, which is not set yet, on an objective portion of a set silicone impression material, and flowing a gypsum paste to the silicone impression material from the upper side of the gum model material. When the gypsum model is taken from the silicone impression material after the gypsum and the gum model material are fully set, a gum is reproduced with the gum model material on the gypsum model.

When such a gum is reproduced, it is important that a gum model material does not adhere with a silicone impression material. However, when a material mainly including silicone is used as a gum model material, the gum model material adheres with a set body of the silicone impression material mainly including silicone. Thus, in the conventional technique, use of a separation material is required. However, when a separation material is used, there are problems that an operation is complicated, a gum model material partially adheres with the silicone impression material mainly including silicone due to the unevenness of coating, and the surface property of a set gum model material becomes inferior due to the separation material. On the other hand, when a material mainly including polyether is used as a gum model material, the material does not adhere with a silicone impression material, but there is a problem that polyether has low storage stability.

In addition to these techniques of using a gum model material, it is known that a model reproducing the impression of oral mucosa can be produced in a short time with high accuracy by taking an impression of an oral cavity with a silicone impression material and then further pouring a silicone composition to the impression material. However, of course, this technique requires use of a separation material. Further, in other fields than the above-described dental field, a technique for producing a complex by building a silicone composition on a set material mainly including silicone is used, but the problem due to a separation material occurs in all these fields.

As mentioned above, a silicone composition, which does not adhere with a set material mainly including silicone when being set on the material without using a separation material, has never been provided in the conventional techniques.

An objective of the present invention is to provide a silicone composition which does not adhere on a set material mainly including silicone, such as a silicone impression material used in the dental operation field, when being built on the set material mainly including silicone.

Present inventors carried out earnest works to solve above-described problems and, as a result, they found out the followings to complete the present invention. The problem can be solved when organohydrogenpolysiloxane having a specific structure which has at least three hydrogen atoms directly bonding to a silicon atom in one molecule and includes SiO₂ units is added to an organopolysiloxane having at least two aliphatic unsaturated hydrocarbons in one molecule as a crosslinking agent, wherein the organopolysiloxane is used as a main component of a conventional dental silicone impression material, fine silica powder having a BET specific surface area of 50 to 500 m²/g and being subjected to a hydrophobic treatment on a surface thereof is further added, and then a silicone-soluble platinum compound in a predetermined amount is added as a catalyst to the material.

That is, the present invention is a silicone composition including (A) 100 weight parts of organopolysiloxane having at least two aliphatic unsaturated hydrocarbons in one molecule, (B) 0.1 to 50 weight parts of organohydrogenpolysiloxane having at least three hydrogen atoms, which directly bond to a silicon atom, in one molecule and including SiO₂ units, (C) 0.1 to 50 weight parts of fine silica powder having a BET specific surface area of 50 to 500 m²/g and being subjected to a hydrophobic treatment on the surface thereof, and (D) 10 to 800 ppm of a silicone-soluble platinum compound with respect to 100 weight parts of the total of the (A) and (B) components.

A silicone composition according to the present invention is an excellent silicon composition, which does not adhere with a set material mainly including silicone when it is set on the set material mainly including silicone.

The (A) component of a silicone composition according to the present invention, which is organopolysiloxane having at least two aliphatic unsaturated hydrocarbons in one molecule, is preferably in the linear chain state and is terminated with vinylsilyl groups at both ends of a molecular chain. In this case, a vinyl group at each end can be plural, and a vinyl group can be contained in the molucular chain. Further, while an organosiloxane molecular chain includes an alkyl group, such as a methyl group and an ethyl group, a phenyl group, and a tolyl group, a methyl group and a phenyl group are especially desirable.

In the present invention, the (B) component which is organohydrogenpolysiloxane having at least three hydrogen atoms, which directly bond to a silicon atom, in one molecule and including SiO₂ units is used as a crosslinking agent for the organopolysiloxane. When the crosslinking agent having this specific structure is used together with the (C) component described below, a composition mainlyincludingsiliconecan haveanexcellent property that the composition does not adhere to a set body of a silicone material when being used by building on a set body of the silicone material. Organohydrogenpolysiloxane has ordinarily a simple linear chain structure but includes SiO₂ units in the present invention. As for the content of the organohydrogenpolysiloxane having at least three hydrogen atoms, which directly bond to a silicon atom, in one molecule and including SiO₂ units, in case of the composition being divided into two or more components at the time of use, the content is 0.1 to 50 weight parts per each component with respect to 100 weight parts of the (A) component. When the content is less than 0.1 weight parts, setting speed of the silicone composition is remarkably slow. When the content is more than 50 weight parts, the set body of a silicone composition becomes brittle. More preferably, the content is 1 to 20 weight parts.

The (C) composition of the present invention is fine silica powder having a BET specific surface area of 50 to 500 m²/g and being subjected to a hydrophobic treatment on the surface thereof. The fine silica powder has a BET specific surface area of 50 to 500 m²/g, and is subjected to a hydrophobic treatment on the surface thereof. The hydrophobic silica as the (C) component can be obtained by, for example, subjecting fumed silica, which is hydrophilic silica, to a heat treatment with a surface treating agent such as methyltrichlorosilane, dimethyldichlorosilane, trimethylchlorosilane, alkoxysilane corresponding to those, octamethylcyclotetrasiloxane, hexamethyldisiloxane, hexamethyldisilane, or a mixture of those, or those surface treating agents with water. Publicly known hydrophobic silica, in which the whole or most parts of an active silanol group on the surface of the hydrophobic silica is terminated with a hydrophobic group such as (CH₃) ₃SiO_{1/2} units, (CH₃) ₂SiO_{2/2} units, or CH₃SiO_{3/2} units, can be used. Further, in a hydrophobic treatment, after silica is treated with the surface treating agent, the silica can be treated with the same or different surface treating agent. As described above, when the (C) component is used together with the (B) component, such the property that a composition mainly including silicone does not adhere with a set body of a silicone material when being used on the set body is given to the silicone composition according to the present invention, and thus the (C) component is very important.

The fine silica powder as the (C) component is required to have a BET specific surface area of 50 to 500 m²/g. When the BET specific surface area is smaller than 50 m²/g, the silica may partially adhere with the set body of a silicone composition. When the BET specific surface area is larger than 500 m²/g, viscosityof a silicone composition increases, and thus operativity decreases.

As for a content of the (C) component, in case of the composition being divided into two or more components at the time of use, the content is 0.1 to 50 weight parts per each component with respect to 100 weight parts of the (A) component. When the content is less than 0.1 weight parts, the composition adheres with the set body of a silicone material. When the content is more than 50 weight parts, viscosity of the silicone composition increases, and thus operativity decreases. More preferably, the content is 1 to 30 weight parts.

A silicone-soluble platinum compound as the (D) component is a publicly known additive reaction catalyst, such as chloroplatinic acid, alcohol-modified chloroplatinic acid, and a complex of chloroplatinic acid and olefin. More preferably used is a vinylsiloxane complex of chloroplatinic acid. The blending amount of the (D) component is within the range of 10 to 800 ppm with respect to the total amount of the (A) and (B) components at the time of use, and is preferably 20 to 200 ppm. When the amount is less than 10 ppm, there is a problem that a setting speed becomes too low. When the amount is more than 800 ppm, a setting speed is too high and it is uneconomical. The silicone-soluble platinum compound is preferably dissolved with an alcohol-based, ketone-based, ether-based, or hydrocarbon-based solvent, or polysiloxane oil.

A silicone composition according to the present invention can include an inorganic or organic colorant, an inorganic filler, a non-ion activator, a polyether compound, or a silicone resin within the range that the silicone composition does not lose properties.

### [Examples]

The present invention will be described in detail below with referent to examples, but the present invention is not limited to those.

### <Example>

A base paste and a catalyst paste were produced with the blending ratios described in Table 1. Each component in Table 1 was produced by being vacuum-stirred by a planetary mixer.

**[Table 1]**

| (Weight parts) | | | Example 1 | Example 2 | Example 3 | Example 4 | Comparative example 1 |
|---|---|---|---|---|---|---|---|
| | A) Components | Dimethylpolysiloxane terminated with a methylvinylsiloxy group at both ends of a molecular chain | 100 | 100 | | 100 | 100 |
| | A) Components | Diphenylpolysiloxane terminated with a methylvinylsiloxy group at both ends of a molecular chain | | | 100 | | |
| | B) Components | Organohydrogenpolysiloxane containing 30 mol% of methylhydrogensiloxane units and including SiO₂ units | | | | 7 | |
| | B) Components | Organohydrogenpolysiloxane containing 50 mol% of methylhydrogensiloxane units and including SiO₂ units | 5 | 0.2 | 20 | | |
| Base Paste | C) Components | Hydrophobic fine silica powder having a BET specific surface area of 100 m²/g and the surface thereof is terminated with (CH₃) ₃SiO_{1/2} units | | | 30 | | |
| | C) Components | Hydrophobic fine silica powder having a BET specific surface area of 200 m²/g and the surface thereof is terminated with (CH₃) ₃SiO_{1/2} units | 20 | | | | |
| | C) Components | Hydrophobic fine silica powder having a BET specific surface area of 300 m²/g and the surface thereof is terminated with (CH₃) ₃SiO_{1/2} units | | 1 | | | |
| | | Quartz powders | | | | 50 | 50 |
| | | Linear chain organohydrogenpolysiloxane containing 30mol% of methylhydrogensiloxane units | | | | | 7 |
| | A) Components | Dimethylpolysiloxane terminated with methylvinylsiloxy groups at both ends of a molecular chain | 100 | 100 | | 100 | 100 |
| | A) Components | Diphenylpolysiloxane terminated with methylvinylsiloxy groups at both ends of a molecular chain | | | 100 | | |
| | D) Components | Silicone oil solution containing 0.5% by weight of 1,3 divinyltetramethyldisiloxane-platinum complex | 4 | 1 | 5 | 5 | 5 |
| Catalyst Paste | C) Components | Hydrophobic fine silica powder having a BET specific surface area of 100 m²/g and the surface thereof is terminated with (CH₃) ₃SiO_{1/2} units | | | 30 | | |
| | C) Components | Hydrophobic fine silica powder having a BET specific surface area of 200 m²/g and the surface thereof is terminated with (CH₃) ₃SiO_{1/2} units | 20 | | | 25 | 25 |
| | C) Components | Hydrophobic fine silica powder having a BET specific surface area of 300 m²/g and the surface thereof is terminated with (CH₃) ₃SiO_{1/2} units | | 1 | | | |
| | | | | | | | |
| Amounts of a silicone-soluble platinum compound with respect to the total 100 weight parts of A) and B) components when mixing a base paste and a catalyst paste. (ppm) | | | 98 | 25 | 114 | 121 | 125 |

### <Measuring of Peeling Strength>

A flat plate of 60×60×2 mm was made with a commercial dental silicone impression material (the product name: EXAMIXFINE Regular Type, produced by GC Corporation). A part of 2/3 from one end of the flat plate was covered with a polyethylene sheet. A silicone composition made by mixing the compositions of the examples with a mixing ratio of a base and a catalyst of 1:1 was built on the dental silicone impression material and pressed so as to make a set body having the thickness of 2 mm. Then, a peeling test was carried out by cutting the end of the set body contacting the polyethylene sheet to have the width of approximately 10 mm, and turning the set body by 180°. In this case, the polyethylene sheet was to completely separate the dental silicone impression material and the silicone compositions of the examples, and thereby the dental silicone impression material and the silicone compositions of the examples were partially contacted. Thus, the peeling strength can be easily tested. These results were shown in Table 2.

### <Test of Releasability>

Releasability tests were carried out in order to confirm that a set body of a silicone composition according to the present invention could be smoothly released from a set body of a dental silicone impression material, which reproduced the complicated shape of an oral cavity, and there occurred no problem about the surface property of a portion contacting with the dental silicone impression material. The model of a lower jaw of an oral cavity was obtained by impression with a commercial dental silicone impression material (the product name: EXAMIXFINE REGULAR TYPE, produced by GC Corporation). Then, the silicone compositions of the examples were mixed with a mixing ratio of a base and a catalyst of 1:1, and flowed the mixture to the set dental silicone impression material. After approximately 1 hour, the set silicone composition was removed from the dental silicone impression material, and the condition of the surface of the silicone composition contacting with the surface of the dental silicone impression material was visually observed. These results were shown in Table 2.

### <Comparative example 2>

The same tests as those in Example 1 were carried out using a commercial dental silicone impression material (the product name: EXAHIFLEX REGULAR, produced by GC Corporation) .

**[Table 2]**

| | Examples | | | | Comparative examples | |
|---|---|---|---|---|---|---|
| | 1 | 2 | 3 | 4 | 1 | 2 |
| Peeling Strength (N/m) | Composition was peeled when a test piece is turned. (before measuring) | Composition was peeled when a test piece is turned. (before measuring) | Composition was peeled when a test piece is turned. (before measuring) | Composition was peeled when a test piece is turned. (before measuring) | 2.6 (completely adhered) | 1.6 (completely adhered) |
| Releasability with a silicone impression material | Composition did not adhere with a silicone impression material and has a smooth surface contacting with a silicone impression material. | Composition did not adhere with a silicone impression material and has a smooth surface contacting with a silicone impression material. | Composition did not adhere with a silicone impression material and has a smooth surface contacting with a silicone impression material. | Composition did not adhere with a silicone impression material and has a smooth impression surface contacting with a silicone impression material. | Composition adhered completely with a silicone material, and could not be removed | Composition adhered completely with a silicone impression material, and could not be removed |

As shown in Table 2, the silicone compositions according to the present invention shown in the examples do not adhere with a set body of a dental silicone impression material when being built on the set body, and peeled before the peeling test. Further, the silicone compositions do not adhere even with that having a complicated shape such as the inside of an actual oral cavity. When the silicone compositions are built and set on a set body of a set dental silicone impression material, the compositions can be removed from the impression material without any problems. On the other hand, certain force is needed to peel the composition of comparative examples. Further, the compositions completely adhered with the complicated shape in an oral cavity, and thus cannot be removed. Accordingly, the silicone composition according to the present invention does not adhere with a set body of a material mainly including silicone such as a dental impression material even when being built and set on the set body.

## Claims

1. A silicone composition comprising:
(A) 100 weight parts of organopolysiloxane having at least two aliphatic unsaturated hydrocarbons in one molecule;
(B) 0.1 to 50 weight parts of organohydrogenpolysiloxane having at least three hydrogen atoms, which directly bond to a silicon atom, in one molecule and including SiO₂ units;
(C) 0.1 to 50 weight parts of fine silica powder having a BET specific surface area of 50 to 500 m²/g and being subjected to a hydrophobic treatment on the surface thereof; and
(D) 10 to 800 ppm of a silicone-soluble platinum compound with respect to 100 weight parts of the total of the (A) and (B) components.
